(19) Europäisches Patentamt
European Patent Office
Office européen des brevets

(11) Numéro de publication: **0 240 530**
**B1**

# (12) FASCICULE DE BREVET EUROPEEN

(45) Date de publication du fascicule du brevet: **25.04.90**

(51) Int. Cl.$^5$: **C 07 C 55/07**, G 11 B 5/706

(21) Numéro de dépôt: **86905854.5**

(22) Date de dépôt: **30.09.86**

(86) Numéro de dépôt international:
**PCT/FR86/00338**

(87) Numéro de publication internationale:
**WO 87/02033 09.04.87 Gazette 87/08**

## (54) COMPOSITIONS PARTICULAIRES D'OXALATES DE METAUX FERROMAGNETIQUES, SOUS FORME DE PARTICULES ACICULAIRES SUBMICRONIQUES, LEUR PREPARATION ET LEUR APPLICATION.

(30) Priorité: **30.09.85 FR 8514438**

(43) Date de publication de la demande:
**14.10.87 Bulletin 87/42**

(45) Mention de la délivrance du brevet:
**25.04.90 Bulletin 90/17**

(84) Etats contractants désignés:
**AT BE CH DE FR GB IT LI LU NL SE**

(56) Documents cités:
**EP-A-0 102 881**
**FR-A-2 284 580**
**US-A-2 636 892**
**US-A-3 317 574**
**US-A-4 004 917**
**US-A-4 170 602**

(73) Titulaire: **CENTRE NATIONAL DE LA RECHERCHE SCIENTIFIQUE (CNRS)**
**15, Quai Anatole France**
**F-75007 Paris (FR)**
(73) Titulaire: **UNIVERSITE PAUL SABATIER (TOULOUSE III)**
**118 Route de Narbonne**
**F-31077 Toulouse Cédex (FR)**

(72) Inventeur: **ROUSSET, Abel**
**16, rue Jean Moulin**
**F-31520 Ramonville St Agne (FR)**
Inventeur: **BONINO SALVAING, Christiane**
**119, rue Achille Viadieu**
**F-31400 Toulouse (FR)**
Inventeur: **MOLLARD, Paul**
**Chemin de la Briot Murianette**
**F-38420 Domène (FR)**
Inventeur: **MANOUX, Alain**
**7, rue Paul Cézanne, A 15024**
**F-91100 Corbeil Essonnes (FR)**
Inventeur: **TAILHADES, Philippe**
**25, rue du Fort**
**F-81200 Mazamet (FR)**

(74) Mandataire: **Tonnellier, Jean-Claude et al**
**Cabinet Nony & Cie. 29, rue Cambacérès**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 240 530 B1

Courier Press, Leamington Spa, England.

**Description**

La présente invention a pour objet des compositions particulaires d'oxalates de métaux ferromagnétiques, sous forme de particules aciculaires submicroniques, leur préparation et leur application notamment comme produits intermédiaires dans la préparation de poudres magnétiques.

On sait que l'industrie de la fabrication des milieux pour l'enregistrement magnétique (bandes, pistes, disques, cartes magnétiques, etc...) utilise des poudres magnétiques d'oxydes, d'alliages, ou d'autres matériaux dérivés de métaux.

Ces poudres peuvent être obtenues au départ de sels organiques, et en particulier au départ d'oxalates; voir par exemple le brevet US 3.317.574 et le brevet US 3.994.819.

On sait que l'on peut caractériser une particule par ses dimensions dans les sens longitudinal (longueur) et transversal (diamètre), et aussi par le rapport aciculaire, qui est le rapport de la longueur au diamètre.

On admet généralement que, dans le domaine d'utilisation mentionné ci-dessus, les meilleurs résultats sont obtenus avec des particules dont la longueur est comprise entre 0,05 et 0,5 micromètre et le rapport aciculaire est compris entre 2 et 5 environ.

La densité des informations enregistrées dépend en effet de la dimension des particules, et les valeurs indiquées ci-dessus correspondent à des valeurs optimales puisque, pour des valeurs plus faibles, les phénomènes de superparamagnétisme peuvent intervenir et diminuer les performances.

Dans le brevet US 3.317.574, on a décrit un procédé consistant à prépare une solution aqueuse d'un mélange d'un sel minéral d'un métal ferromagnétique et d'un acide carboxylique, puis à ajouter à cette solution un solvant organique capable de provoquer la précipitation des sels d'acide organique des métaux présents.

Un tel procédé ne permet toutefois d'obtenir que des particules ayant un rapport aciculaire voisin de 2 pour les particules submicroniques, ce qui est insuffisant pour certaines applications. Avec le même procédé, il est possible d'obtenir des particules ayant un rapport aciculaire plus élevé, mais uniquement pour des particules ayant une longueur moyenne supérieure à 1 micromètre.

Dans le brevet US 3.994.819, on a décrit un procédé permettant d'obtenir, par addition lente d'une solution hydroalcoolique de sels minéraux de métaux ferromagnétiques dans une solution hydroalcoolique d'acide oxalique, des particules d'oxalates ayant une longueur moyenne généralement comprise entre 0,5 et 1 micromètre, avec un rapport aciculaire relativement faible. Dans le même brevet US, on a décrit un procédé permettant de diminiuer la longueur des particules, ce procédé consistant à ajouter très lentement la solution de sels à la solution d'acide oxalique dans un mélange d'éthanol et d'éther de pétrole, et en effectuant l'étape de précipitation à − 20°C. On obtient des particules de longueur moyenne égale à 0,4—0,5 micromètre, et le rapport aciculaire moyen est égal à 12 environ, ce qui est un peu trop élevé. Mais surtout, les particules obtenues sont trop hétérogènes pour certaines applications.

En outre, ce procédé ne permet pas d'utiliser le sulfate ferreux comme produit de départ: il est nécessaire d'utiliser au moins en partie du chlorure ferreux, plus soluble mais plus coûteux, pour éviter d'opérer sur des volumes de solution excessifs.

La présente invention repose sur la découverte d'un nouveau procédé qui a permis d'obtenir pour la première fois des particules d'oxalates simples ou mixtes de métaux ferromagnétiques ayant des dimensions et des rapports aciculaires conformes aux normes optimales rappelées ci-dessus. Le procédé de l'invention présente en outre l'avantage de ne pas nécessiter de refroidissement, de permettre l'utilization exclusive de sulfate ferreux, et d'être plus rapide que les procédé antérieurs.

La présente invention a donc pour objet une composition particulaire d'oxalate simple ou mixte de métaux ferromagnétiques, caractérisée par le fait qu'elle est constituée de particules aciculaires ayant une longueur allant de 0,05 à 0,5 micromètre, avec plus de 60% des particules ayant une longueur égale à la longueur moyenne ± 0,1 micromètre et un rapport aciculaire supérieur à 3, ladite longueur moyenne étant comprise entre 0,15 et 0,35 micromètre.

Le procédé de l'invention permet même de préparer dans certains cas des compositions particulaires d'oxalates dont plus de 80% des particules, et même parfois plus de 90% des particules, présentent ces caractéristiques. C'est cette grande homogénéité des particules qui constitue l'une des caractéristiques les plus importantes de l'invention.

Les particules d'oxalate faisant l'objet de l'invention peuvent encore présenter les caractéristiques suivantes, prises isolément ou en combinaison:

Le rapport aciculaire moyen des particules varie par exemple de 3 à 10:

Le métal ferromagnétique est choisi parmi le fer, le cobalt et le nickel;

Ledit oxalate est un oxalate mixte comprenant, outre au moins un métal ferromagnétique, au moins un métal bivalent choisi parmi le cuivre, le zinc, le cadmium, le magnésium, le manganèse, les métaux alcalino-terreux (calcium, baryum, strontium), l'europium, l'ytterbium, l'étain et le plomb;

Ledit oxalate contient en outre, à titre d'agent adjuvant ou dopant, un ou plusieurs éléments choisis parmi le lithium, le potassium, l'aluminium, le scandium, le titane, le vanadium, le chrome, l'yttrium, le zirconium, le niobium, le molybdène, le bismuth, l'arsenic, l'antimoine, le silicium, le germanium, le bore, le gallium, l'indium et les terres rares trivalentes (par exemple gadolinium, néodyme, dysprosium, samarium, terbium, cérium, praséodyme, holmium, erbium, thulium, lutétium); la proportion d'agent

dopant est fonction de l'utilisation envisagée pour le produit final et est généralement inférieure à 3% en poids;

Ledit oxalate est un oxalate à base de fer contenant au moins 60% d'atomes de fer par rapport à la totalité des atomes métalliques;

Ledit oxalate correspond (les agents dopants éventuels mis à part), à la formule:

$$(Fe_{1-x}M_x)C_2O_4,\ yH_2O$$

dans laquelle:

M représente au moins un métal divalent choisi parmi le cobalt, le nickel, le cuivre, le zinc, le cadmium, le magnésium, le manganèse, les métaux alcalino-terreux, l'europium, l'ytterbium, l'étain et le plomb,

y est un nombre supérieur ou égal à zéro et inférieur ou égal à 2,

et x est un nombre inférieur ou égal à 1,3.

Le plus souvent, x est inférieur à 0,15.

L'invention a également pour objet un procédé de préparation d'une masse particulaire d'oxalate, telle que définie précédemment, ce procédé étant caractérisé par le fait:

que, d'une part, on prépare une solution contenant de 0,1 à 1 mole par litre d'acide oxalique dans un premier solvant organique ayant une constante diélectrique inférieure à 30, ladite solution pouvant contenir au maximum 15% en volume d'eau;

que, d'autre part, on prépare une solution concentrée d'au moins un sel minéral du métal ou des métaux que l'on désire obtenir sous forme d'oxalate, dans un milieu liquide contenant au moins 25% en volume d'un second solvant organique choisi parmi le méthanol, l'éthanol, le tétrahydrofuranne, et les polyols liquides à la température ambiante, ou des mélanges de ces solvants, le reste éventuel dudit milieu liquide étant constitué par de l'eau, la concentration totale des sels étant supérieure à 1 mol/litre, ladite solution étant acidifiée pour favoriser la solubilisation des sels et contenant en outre les agents dopants éventuels;

puis que l'on verse progressivement ladite solution de sel dans ladite solution d'acide oxalique en agitant cette dernière, pour obtenir un précipité particulaire d'oxalate;

étant entendu que l'un au moins desdits solvants organiques est un alcool.

L'une des caractéristiques principales du procédé de l'invention est d'opérer en présence d'un minimum d'eau, sans toutefois nécessiter l'emploi de solvants et de réactifs anhydres; par exemple, les sels et l'acide oxalique peuvent être utilisés sous la forme d'hydrates, et l'éthanol utilisé peut être l'alcool commercial à 95% ou même à 90%. C'est un employant le minimum d'eau que l'on a pu obtenir, selon le procédé de l'invention, des particules homogènes aciculaires de dimensions très faibles.

Parmi les solvants organiques capables de solubiliser l'acide oxalique avec un minimum d'eau, ou même sans eau (l'eau de cristallisation de l'acide oxalique exceptée), on citera notamment l'éthanol, le propanol-1, l'isopropanol, le butanol, l'acétone, l'éther éthylique, les mélanges éther de pétrole/éthanol, ou encore des mélanges de ces solvants. Les solvants ayant les constantes diélectriques les plus faibles donneront des particules de dimensions plus réduits.

Pour solubiliser les sels de départ, il est généralement nécessaire d'utiliser un milieu aqueux, mais on diminue la quantité d'eau d'une part par l'addition du second solvant organique et d'autre part en acidifiant le milieu avec un acide tel que l'acide chlorhydrique, l'acide sulfurique, l'acide phosphorique, l'acide acétique, etc..

Bien entendu, l'acide utilisé ne doit pas donner de sel insoluble avec les métaux des sels de départ. En outre, l'acide stabilise les sels ferreux s'ils sont présents. Dans certains cas, il est possible de solubiliser les sels pratiquement sans eau, mise à part l'eau de constitution des sels et l'eau présente dans les solvants organiques commerciaux tels que l'éthanol. On peut utiliser notamment un mélange propanol-méthanol (voir exemple 33).

Lorsqu'on souhaite obtenir des particules de dimensions très faibles, par exemple inférieures à 0,3μ, il est souhaitable de choisir le second solvant organique (présent dans la solution de sels) ayant la plus faible constante diélectrique possible, compatible cependant avec l'obtention d'une solution concentrée de sels. De préférence, l'eau ne représente pas plus de 70% en volume de la solution de sels de départ.

L'effet de la constante diélectrique des solvants est illustré notamment par les exemples 34 et 36—38 ci-après.

Les solvants utilisés pour le sel et pour l'acide oxalique sont généralement différents. Toutefois, on peut utiliser par exemple l'éthanol dans les deux cas.

Comme indiqué ci-dessus, l'un au moins desdits premier et second solvants organiques est un alcool (mono-alcool ou polyol), car cela favorise l'obtention de particules aciculaires.

Compte tenu des indications précédentes, l'invention a également pour objet un procédé, tels que défini ci-dessus, dans lequel on règle la dimension des particules par le choix des solvants, les dimensions des particules étant d'autant plus réduites que la constante diélectrique des solvants organiques est plus faible et que l'eau est absente ou présente en faibles quantités.

La quantité d'acide oxalique utilisée est au moins égale à la quantité stoechiométrique. On préfère généralement utiliser un léger excès d'acide oxalique, par exemple de l'ordre de 10% en moles, afin de précipiter au maximum les métaux du ou des sels de départ.

L'addition de la solution de sels dans la solution d'acide oxalique doit être progressive. Il faut toutefois éviter des durées trop élevées pour cette addition. On opère généralement en moins de trente minutes, et de préférence en 15 minutes ou moins (par exemple en 5 à 15 minutes).

Selon un mode d'exécution préféré, on agite la solution d'acide oxalique et l'on introduit progressivement la solution de sels dans ladite solution agitée en pulvérisant la solution de sels au-dessus de la solution d'acide oxalique.

La quantité d'agent dopant présente dans les particules d'oxalate obtenues par le procédé de l'invention dépend évidemment de la solubilité de l'agent dopant dans le milieu réactionnel.

Il est facile de déterminer dans chaque cas, par de simples expériences de routine, la quantité d'agent dopant à introduire dans le milieu réactionnel pour obtenir la proportion voulue d'agent dopant dans les particules d'oxalate.

Le procédé de l'invention présente l'avantage de ne pas nécessiter un refroidissement coûteux, ni des durées élevées de précipitation qui, liées à la mise en oeuvre de concentrations relativement faibles, augmentaient fortement le coût des produits préparés selon les procédés antérieurs.

Contrairement au procédé du brevet US 3.994.819, le procédé de la présente invention permet d'utiliser des sels de fer uniquement sous la forme de sulfate ferreux.

En outre, le procédé de la présente demande permet d'opérer à température ambiante, et avec des solutions plus concentrées, ce qui évite l'utilisation de volumes de solution trop importants.

Les polyols utilisables pour prépares les solutions de sels minéraux de départ sont d'une façon générale tous polyols (en particulier diols et triols) liquides à la température ambiante, qui, en combinaison éventuellement avec de l'eau ou avec du méthanol, permettent d'obtenir des solutions suffisamment concentrées desdits sels minéraux, y compris le sulfate ferreux (solutions de concentration supérieure à 1M, et de préférence voisine de 2M).

Parmi les polyols qui peuvent être utilisés dans le procédé de la présente demande, on mentionnera en particulier l'éthylèneglycol, le glycérol, le diéthylèneglycol, le propylèneglycol, etc... ainsi que leurs mélanges, ou encore des mélanges d'au moins un de ces polyols avec d'autres solvants tels qu'un alcanol inférieur.

Parmi les solvants et mélanges de solvants utilisables pour réaliser la solution de sels minéraux, on peut citer:

les mélanges propanol-méthanol;

l'éthanol à 10% d'eau;

les mélanges propanediol-eau;

les mélanges tétrahydrofuranne-eau;

les mélanges eau-éthylèneglycol contenant au moins 25% d'éthylèneglycol, et l'éthylèneglycol pur;

les mélanges contenant au moins 20% d'éthylèneglycol et au moins 20% d'un autre polyol ou de méthanol, le reste étant de l'eau;

les mélanges eau-méthanol contenant au moins 50% de méthanol.

Grâce à l'utilisation de ces solvants, il a été possible d'augmenter la concentration des sels et de diminuer les dimensions des particules obtenues, tout en travaillant à la température ambiante.

En outre, on a constaté, de façon surprenante, qu'il était possible d'augmenter considérablement la vitesse de réaction, sans affecter défavorablement les caractéristiques morphologiques des particules obtenues, en opérant l'introduction de la solution des sels par pulvérisation de cette solution au-dessus de la solution d'acide oxalique convenablement agitée.

La vitesse de réaction peut ainsi être 20 fois supérieure à celle obtenue par la technique antérieure du brevet US 3.994.819.

L'invention a également pour objet l'utilisation des compositions particulaires d'oxalate ainsi obtenues dans la préparation de particules d'oxydes, de nitrures, d'oxynitrures, de borures, de carbonitrures, d'oxycarbonitrures, de métaux ou d'alliages métalliques correspondants qui peuvent être obtenues à partir des oxalates selon les méthodes connues; voir par exemple le brevet britannique 679.172, le brevet US 3.317.574, les brevets français 2.180.575 et 2.284.580, etc...

Les exemples suivants illustrent l'invention sans toutefois la limiter.

### Example 1

On prépare une solution A d'acide oxalique 0,5 molaire en dissolvant 13,75 grammes d'acide oxalique dans de l'alcool éthylique à 95% en volume, à température ambiante.

On prépare une solution B deux fois molaire en dissolvant 26 grammes de sulfate de fer II, 1 gramme de sulfate de cobalt II ($CoSO_4$, $7H_2O$) et 0,7 gramme de sulfate de zinc ($ZnSO_4$, $7H_2O$) dans un mélange renfermant 60% d'eau et 40% d'éthylène glycol auquel on ajoute 0,5 ml de HCl 12N.

Dans la solution A, fortement agitée on introduit par pulvérisation à raison de 7 l/heure la solution B afin d'obtenir un précipité d'oxalate mixtre de Fe, Co, Zn de formule $(Fe_{0,938}Co_{0,038}Zn_{0,024})C_2O_4$, $2H_2O$.

La pulvérisation est effectuée à l'aide d'un pulvérisateur permettant d'obtenir des gouttes ayant des dimensions moyennes de 0,8 mm.

Après lavage et séchage du précipité, l'examen au microscope électronique permet de procéder à la mesure des dimensions et au comptage des particules. On constante que plus de 90% de particules ont une longueur de 0,38 ± 0,08 micromètre et un diamètre de 0,042 ± 0,01 micromètre.

La figure 1 est un cliché de microscopie électronique des particules obtenues.

Exemple 2

On prépare une solution A identique à celle de l'exemple 1.

On prépare une solution B deux fois molaire en dissolvant 25,9 grammes de sulfate de fer II ($FeSO_4,7H_2O$), 0,9 gramme de sulfate de cobalt ($CoSO_4$, $7H_2O$), et 1,2 g de sulfate de zinc ($ZnSO_4$, $7H_2O$) dans un mélange refermant 30% d'eau et 70% de méthanol auquel on ajoute 10 ml d'acide chlorhydrique 12 N.

Dans la solution A, fortement agitée on introduit par pulvérisation à raison de 7 l/heure la solution B afin d'obtenir un précipité d'oxalate mixte de Fe, Co, Zn de formule:

$$(Fe_{0,923}Co_{0,034}Zn_{0,043})C_2O_4,\ 2H_2O$$

Après lavage et séchage du précipité, l'examen au microscope électronique permet de déterminer qu'on a obtenu des particules aciculaires dont plus de 80% ont une longueur de 0,35±0,10 micromètre et un diamètre de 0,05±0,01 micromètre.

La figure 2 est un cliché de microscopie électronique des particules obtenus.

Exemple 3

On prépare une solution A identique à celle des exemples précédents.

On prépare une solution B deux fois molaire en dissolvant 25,9 grammes de sulfate de fer II ($FeSO_4$, $7H_2O$), 0,9 gramme de sulfate de cobalt ($CoSO_4$, $7H_2O$) et 1,2 gramme de sulfate de zinc ($Zn\ SO_4$, $7H_2O$) dans un mélange renfermant 25% d'eau, 25% d'éthylèneglycol et 50% de méthanol, auquel on ajoute 0,5 ml d'acide chlorhydrique 12 N.

Dans la solution A fortement agitée on introduit par pulvérisation, à raison de 7 l/heure, la solution B afin d'obtenir un précipitée d'oxalate mixte de Fe, Co, Zn de formule:

$$(Fe_{0,923}Co_{0,034}Zn_{0,043})\ C_2O_4,\ 2H_2O$$

Après lavage et séchage du précipité, l'examen au microscope électronique permet de déterminer que l'on a obtenu des particules aciculaires dont plus de 80% ont une longueur de 0,38 ± 0,10 micromètre et un diamètre de 0,050±0,015 micromètre.

Exemple 4

On prépare une solution A identique à celle des exemples précédents.

On prépare une solution B deux fois molaire en dissolvant 26 grammes de sulfate de fer II ($FeSO_4$, $7H_2O$) 1 gramme de sulfate de cobalt ($CoSO_4$, $7H_2O$) et 0,7 gramme de sulfate de zinc ($ZnSO_4$, $7H_2O$) dans un mélange refermant 20% d'eau, 40% d'éthyléneglycol et 40% de glycérol, auquel on ajoute 0,5 ml d'acide chlorhydrique 12 N.

Dans la solution A fortement agitée, on introduit par pulvérisation, à raison de 7 l/heure, la solution B afin d'obtenir un précipité d'oxalate mixte de Fe, Co, Zn de formule:

$$(Fe_{0,938}Co_{0,038}Zn_{0,024})\ C_2O_4,\ 2H_2O$$

Après lavage et séchage du précipité, l'examen au microscope électronique permet de déterminer que l'on a obtenu des particules aciculaires dont plus de 85% ont une longueur de 0,37 ± 0,10 micromètre et un diamètre de 0,048 ± 0,015 micromètre.

Des oxalates mixtes aciculaires de dimensions identiques à celles indiquées précédemment peuvent être obtenus en utilisant des chlorures de fer, de cobalt et de zinc à la place des sulfates.

Exemple 5

On opère comme à l'exemple 1, excepté que dans la solution B, on ajoute en outre 1,4 g de $MnCl_2$, $6H_2O$. On obtient des particules dont la composition correspond à la formula:

$$(Fe_{0,872}Co_{0,038}Zn_{0,024}Mn_{0,066})\ C_2O_4,\ 2H_2O$$

Les dimensions des particules sont semblables à celles des particules de l'exemple 1.

Exemple 6

On opéra comme à l'exemple 1, mais en remplaçant le sulfate ferreux par 19,9 g de $FeCl_2$, $4H_2O$, et on ajoute en outre à la solution B 2 g de $BaCl_2$. On obtient des particules d'oxalate répondant à la formule:

$$(Fe_{0,925}Co_{0,038}Zn_{0,024}Ba_{0,013})\ C_2O_4,\ 2H_2O$$

Les dimensions des particules sont semblables à celles des particules de l'exemple 1.

Exemple 7

On opère comme à l'exemple 2, mais on ajoute en outre à la solution B 0,16 g de $Eu(NO_3)_2$. On obtient des particules d'oxalate de formule:

$$(Fe_{0,915}Co_{0,034}Zn_{0,043}Eu_{0,008})\ C_2O_4,\ 2H_2O$$

Les dimensions des particules sont semblables à celles des particules de l'exemple 2.

Exemple 8

On opére comme à l'exemple 1, mais on ajoute en outre à la solution B 0,45 g de $TiCl_3$. On obtient des particules d'oxalate correspondant (le dopant oxalate de titane mis à part) à la formule donnée à l'exemple 1. Ces particules contiennent 0,25% en poids de titane.

Les dimensions des particules sont semblables à celles des particules de l'exemple 1.

Exemple 9

On opère comme à l'exemple 1, mais on ajoute en outre à la solution B 3g de $SbCl_3$. On obtient des particules d'oxalate correspondant (le dopant oxalate d'antimoine mis à part) à la formule donnée à l'exemple 1. Ces particules contiennent 0,15% en poids d'antimoine.

Les dimensions des particules sont semblables à celles des particules de l'exemple 1.

Exemple 10

On opère comme à l'exemple 1, mais on ajoute en outre à la solution B 0,45 g de $TiCl_3$ et 1,5 g d'$AlCl_3$. On obtient des particules d'oxalate correspondant (le dopant oxalate de titane et d'aluminium mis à part) à la formule donnée à l'exemple 1. Ces particules contiennent 0,05% en poids de titane et 0,7% d'aluminium.

Les dimensions des particules sont semblables à celles des particules de l'exemple 1.

Exemple 11

On opère comme à l'exemple 1, mais on ajoute en outre à la solution B 0,5 g de $VCl_3$. On obtient des particules d'oxalate correspondant (le dopant oxalate de vanadium mis à part) à la formule donnée à l'exemple 1. Ces particules contiennent 1,2% en poids de vanadium.

Les dimensions des particules sont semblables à celles des particules de l'exemple 1.

Exemple 12

On opère comme à l'exemple 1, mais on ajoute en outre à la solution B 0,3 g de KCl. On obtient des particules d'oxalate correspondant (le dopant oxalate de potassium mis à part) à la formule donnée à l'exemple 1. Ces particules contiennent 0,3% en poids de potassium.

Les dimensions des particules sont semblables à celles des particules de l'exemple 1.

Exemple 13

On opère comme à l'exemple 2, mais on ajoute en outre à la solution B 0,5 g de $YbCl_2$. On obtient des particules d'oxalate correspondant (le dopant oxalate d'ytterbium mis à part) à la formule donnée à l'exemple 2. Ces particules contiennent 2% en poids d'ytterbium.

Les dimensions des particules sont semblables à celles des particules de l'exemple 2.

Exemple 14

On opère comme à l'exemple 2, mais on ajoute en outre à la solution B 0,5 g de $GdCl_3$, $6H_2O$. On obtient des particules d'oxalate correspondant (le dopant oxalate de gadolinium mis à part) à la formule donnée à l'exemple 2. Ces particules contiennent 2,8% en poids de gadolinium.

Les dimensions des particules sont semblables à celles des particules de l'exemple 2.

Exemple 15

On opère comme à l'exemple 2, mais on ajoute en outre à la solution B 0,5 g de $DyCl_3$. On obtient des particules d'oxalate correspondant (le dopant oxalate de dysprosium mis à part) à la formule donnée à l'exemple 2. Ces particules contiennent 2,7% en poids de dysprosium.

Les dimensions des particules sont semblables à celles des particules de l'exemple 2.

Exemple 16

On prépare une solution A d'acide oxalique 0,5 molaire en dissolvant 13,75 g d'acide oxalique dans de l'alcool éthylique à 95% en volume, à température ambiante.

On prépare une solution B deux fois molaire en dissolvant, dans un mélange contenant 60% d'eau et 40% d'éthylèneglycol, les sels suivants:

$FeCl_2$, $4H_2O$ : 18,7 g
$CoCl_2$, $6H_2O$ : 1 g
$ZnCl_2$ : 0,5 g
$BaCl_2$ : 0,8 g

On ajoute au mélange 0,5 $cm^3$ d'une solution aqueuse d'acide chlorhydrique 12N. On introduit la solution B dans la solution A fortement agitée, par pulvérisation au dessus de la solution A, à raison de 7 litres/h, de la solution B, à l'aide d'un pulvérisateur permettant d'obtenir des gouttes de 0,8 mm en moyenne.

On lave et sèche les particules d'oxalate qui ont précipité.

On obtient des particules d'oxalante de formule:

$$(Fe_{0,9391}Co_{0,0420}Zn_{0,0124}Ba_{0,0064})^{2+}C_2O_4^{2-}2H_2O.$$

Exemples 17 à 29

De façon analogue, on a préparé des compositions particulaires d'oxalates ayant les compositions suivantes:

Exemple 17

$$(Fe_{0,9393}Co_{0,0430}Zn_{0,0147}Ba_{0,030})^{2+}C_2O_4^{2-}2H_2O.$$

Exemple 18

$$(Fe_{0,9335}Co_{0,0433}Zn_{0,0108}Ba_{0,0124})^{2+}C_2O_4^{2-}2H_2O.$$

Exemple 19

$$(Fe_{0,9450}Co_{0,0437}Ba_{0,0112})^{2+}C_2O_4^{2-}2H_2O.$$

Exemple 20

$$(Fe_{0,9342}Co_{0,0435}Zn_{0,0112}Ba_{0,0111})^{2+}C_2O_4^{2-}2H_2O.$$

Exemple 21

$$(Fe_{0,9300}Co_{0,0437}Zn_{0,0263})^{2+}_{0,9929}Dy^{3+}_{0,0047}C_2O_4^{2-}2H_2O.$$

Exemple 22

$$(Fe_{0,9368}Co_{0,0432}Zn_{0,0135}Ba_{0,0065})^{2+}_{0,9907}Dy^{3+}_{0,9062}C_2O_4^{2-}\ 2H_2O.$$

Exemple 23

$$(Fe_{0,9253}Co_{0,0442}Zn_{0,0264}Yb_{0,0041})^{2+}C_2O_4^{2-}2H_2O.$$

Exemple 24

$$(Fe_{0,9296}Co_{0,0432}Zn_{0,0266})^{2+}_{0,9927}Gd^{3+}{}_{0,0049}C_2O_4^{2-}2H_2O.$$

Exemple 25

$$(Fe_{0,9147}Co_{0,0482}Zn_{0,0334}Eu_{0,00375})^{2+}C_2O_4^{2-}2H_2O.$$

Exemple 26

$$(Fe_{0,9237}Co_{0,0438}Zn_{0,0325})^{2+}_{0,9972}Sb^{3+}_{0,0018}C_2O_4^{2-}2H_2O.$$

Exemple 27

$$(Fe_{0,9336}Co_{0,0390}Zn_{0,0274})^{2+}_{0,9688}V^{3+}_{0,0131}Ti^{4+}_{0,0058}C_2O_4^{2-}2H_2O.$$

Exemple 28

$$(Fe_{0,9324}Co_{0,0456}Zn_{0,0221})^{2+}_{0,9561}\ Ga^{3+}_{0,0216}Ti^{4+}_{0,0058}\ C_2O_4^{2-}2H_2O.$$

Exemple 29

$$(Fe_{0,9438}Co_{0,0412}Zn_{0,0150})^{2+}_{0,9500}\ Al^{3+}_{0,0334}\ C_2O_4^{2-}2H_2O.$$

Dans ces exemples, le dysprosium est introduit sous forme de $DyCl_3$; l'ytterbium sous forme de $YbCl_2$; le gadolinium sous forme de $GdCl_3$; l'europium sous forme de Eu $(No_3)_2$; l'antimoine sous forme de $SbCl_3$; le titane sous forme de $TiCl_3$; le vanadium sous forme de $VCl_3$; le gallium sous forme de $Ga_2(So_4)_3$; et l'aluminium sous forme d'$AlCl_3$.

En outre, les particules d'oxalate de l'exemple 20 ont été dopées au bore par mise en contact avec une solution aqueuse à 3% en poids d'acide borique, à raison de 500 ml de cette solution pour 15 g d'oxalate.

On a obtenu un oxalate correspondant à la formule:

$$(Fe_{0,9342}\ Co_{0,0435}\ Zn_{0,0112}\ Ba_{0,0111})^{2+}C_2O_4,\ ^{2-}2H_2O+0,0249 \text{ mole de } H_3BO_3$$

Exemple 30 — Oxalate de fer

On prépare une solution A d'acide oxalique 0,5 molaire en dissolvant 13,75 g d'acide oxalique dans de l'alcool éthylique à 95% en volume, à température ambiante.

On prépare une solution B deux fois molaire en dissolvant 27,8 g de sulfate de fer II ($FeSO_4$, $7H_2O$) dans un mélange renfermant 60% d'eau et 40% de propanediol-1,2 auquel on ajoute 1 ml de HCl 12N.

Dans la solution A fortement agitée on introduit au goutte à goutte à raison de 0,8 litre/heure la solution B afin d'obtenir un précipité d'oxalate de fer.

Après lavage et séchage du précipité, l'examen au microscope électronique permet de déterminer que l'on a obtenu des particules aciculaires dont plus de 60% ont une longueur de 0,27 ± 0,1 µm et un diamètre de 0,06 µm ± 0,02 µm et un rapport aciculaire moyen de 5,2.

Exemple 31 — Oxalate mixte

On prépare une solution A identique à celle de l'exemple précédent.

On prépare une solution B deux fois molaire en dissolvant 18,4 g de $FeCl_2$, $4H_2O$, 0,96 g de $CoCl_2$, $6H_2O$, 0,45 g de $ZnCl_2$ et 0,8 g de $BaCl_2$ dans un mélange renfermant 60% d'eau et 40% de propanediol-1,2 auquel on ajoute 1 ml HCl 12N.

Dans la solution A on ajoute comme précédemment la solution B afin d'obtenir un précipité d'oxalate mixte de formule:

$$[Fe_{0,937}\ Co_{0,043}\ Zn_{0,013}\ Ba_{0,07}]C_2O_4,\ 2H_2O$$

Après lavage et séchage du précipité, l'examen au microscope électronique permet de déterminer que l'on a obtenu des particules aciculaires dont plus de 80% ont une longueur de 0,25 ± 0,1 µm et 60% ont une diamètre de 0,075 ± 0,02 µm, le rapport aciculaire moyen étant de 3,6.

Exemple 32 — Oxalate de fer

On prépare une solution A identique à celle de l'exemple précédent.

On prépare une solution B deux fois molaire en dissolvant 19,9 g de $FeCl_2$, $4H_2O$ dans un mélange renfermant 60% d'eau et 40% de propanediol-1,2, auquel on ajoute 0,5 ml de HCl 12N.

Dans la solution A on ajoute comme précédemment la solution B afin d'obtenir un précipité d'oxalate de fer.

Après lavage et séchage du précipité, l'examen au microscope électronique permet de déterminer que l'on a obtenu des particules aciculaires dont plus de 60% ont une longueur de 0,26 ± 0,1 µm et un diamètre de 0,043 ± 0,02 µm, le rapport aciculaire moyen étant de 6,4.

Exemple 33 — Oxalate de fer

On prépare une solution A identique à celle de l'exemple précédent.

On prépare une solution B deux fois molaire en dissolvant 19,9 g de $FeCl_2$, $4H_2O$ dans un mélange renfermant 50% de propanol-1 et 50% de méthanol, auquel on ajoute 1,5 ml d'acide chlorhydrique 12N.

Dans la solution A, on ajoute comme précédemment la solution B afin d'obtenir un précipité d'oxalate de fer.

Après lavage et séchage du précipité l'examen au microscope électronique permet de déterminer que l'on a obtenu des particules aciculaires dont plus de 60% ont une longueur de 0,20 ± 0,1 µm et un diamètre de 0,06 ± 0,02 µm, le rapport aciculaire moyen étant de 3,7.

Exemple 34 — Oxalate de fer

On prépare une solution A d'acide oxalique 0,5 molaire en dissolvant 13,75 g d'acide oxalique dans du butanol-1 à température ambiante.

On prépare une solution B 1,5 fois molaire en dissolvant 19,9 g de $FeCl_2$, $4H_2O$ dans un mélange renfermant 90% d'éthanol et 10% d'eau auquel on ajoute, 1,5 ml de HCl 12N.

Dans la solution A, on ajoute comme précédemment la solution B afin d'obtenir un précipité d'oxalate de fer.

Après lavage et séchage du précipité, l'examen au microscope électronique permet de déterminer que l'on a obtenu des particules aciculaires dont plus de 70% ont une longueur de 0,17 ± 0,1 µm et un diamètre de 0,04 ± 0,02 µm, le rapport aciculaire moyen étant de 4,6.

Exemple 35 — Oxalate de fer

On prépare une solution A identique à celle de l'exemple 1.

On prépare une émulsion B deux fois molaire en dissolvant 19,9 g de $FeCl_2$, $4H_2O$ dans un mélange renfermant 60% d'eau et 40% de tétrahydrofuranne (THF) auquel on ajoute 0,5 ml de HCl 12N.

On ajoute comme précédemment la solution B à la solution A afin d'obtenir un précipité d'oxalate de fer.

Après lavage et séchage du précipité l'examen au microscope électronique permet de déterminer que l'on a obtenu des particules aciculaires dont plus de 70% ont une longueur de 0,22 ± 0,1 µm et un diamètre de 0,045 µm ± 0,02 µm, le rapport aciculaire moyen étant de 5,2.

Exemple 36

En opérant comme à l'exemple 34, mais en remplaçant le butanol (comme solvant de l'acide oxalique) par l'éthanol, on obtient des particules d'oxalate de fer de longueur moyenne 0,27 ± 0,1 µm et de diamètre moyen 0,038 µm.

Exemple 37

En opérant comme à l'exemple 34, mais en remplaçant le butanol par le propanol-1, on obtient des particules d'oxalate de fer de longueur moyenne 0,24 ± 0,1 µm et de diamètre moyen 0,055 µm.

Exemple 38

En opérant comme à l'exemple 34, mais en remplaçant, dans la solution de sel, l'éthanol par le propanediol (autrement dit, on utilise un mélange propanediol-eau à 90% de propanediol).

On obtient des particules d'oxlate de fer ayant une longueur moyenne de 0,31 ± 0,1 µm et un diamètre moyen de 0,046 µm.

**Revendications**

1. Composition particulaire d'oxalate simple ou mixte de métaux ferromagnétiques, caractérisée par le fait qu'elle se présente sous la forme de particules aciculaires ayant une longueur de 0,05 à 0,5 micromètre, avec une proportion de plus de 60% des particules ayant une longueur égale à la longueur moyenne ± 0,1 µm, et un rapport aciculaire supérieur à 3, ladite longueur moyenne étant comprise entre 0,15 et 0,35 µm.

2. Composition particulaire selon la revendication 1, caractérisée par le fait que ladite proportion est supérieure à 80%.

3. Composition particulaire selon la revendication 1, caractérisée par le fait que le métal ferromagnétique est choisi parmi le fer, le cobalt et le nickel.

4. Composition particulaire selon l'une quelconque des revendications précédentes, caractérisée par le fait que ledit oxalate est un oxalate mixtre comprenant, outre au moins un métal ferromagnétique, au moins un métal bivalent choisi parmi le cuivre, le zinc, le cadmium, le magnésium, le manganèse, les métaux alcalino-terreux (calcium, baryum, strontium), l'europium, l'ytterbium, l'étain et le plomb.

5. Composition particulaire selon l'une quelconque des revendications précédentes, caractérisée par le fait que ledit oxalate contient en outre, à titre d'agent adjuvant ou dopant, un ou plusieurs éléments choisis parmi le lithium, le potassium, l'aluminium, le scandium, le titane, le vanadium, le chrome, l'yttrium, le zirconium, le niobium, le molybdène, le bismuth, l'arsenic, le bore, l'antimoine, le silicium, le germanium, le gallium, l'indium, les terres rares trivalentes (par exemple gadolinium, néodyme, dysprosium, samarium, terbium, cérium, praséodyme, holmium, erbium, thulium, lutétium).

6. Composition particulaire selon la revendication 5, caractérisée par le fait que ledit agent dopant est présent dans une proportion inférieure à 3% en poids.

7. Composition particulaire selon l'une quelconque des revendications précédentes, caractérisée par le fait que ledit oxalate est un oxalate à base de fer contenant au moins 60% d'atomes de fer par rapport à la totalité des atomes métalliques.

8. Composition particulaire selon l'une quelconque des revendications précédentes, caractérisée par le fait que ledit oxalate correspond (les agents dopants éventuels mis à part) à la formule:

$$(Fe_{1-x}M_x)\ C_2O_4,\ y\ H_2O$$

dans laquelle:

M représente au moins un métal choisi parmi le cobalt, le nickel, le cuivre, le zinc, le cadmium, le magnésium, le manganèse, les métaux alcalino-terreux, l'europium, l'ytterbium, l'étain et le plomb,

y est un nombre supérieur ou égal à zéro et inférieur ou égal à 2,

et x est un nombre inférieur ou égal à 1,3.

9. Composition particulaire selon la revendication 8, caractérisée par le fait que x est inférieur à 0,15.

10. Procédé de préparation d'une composition particulaire telle que définie dans l'une quelconque des revendications précédentes, caractérisé par le fait:

que, d'une part, on prépare une solution contenant de 0,1 à 1 mole par litre d'acide oxalique dans un premier solvant organique ayant une constante diélectrique inférieure à 30, ladite solution pouvant contenir au maximum 15% en volume d'eau;

que, d'autre part, on prépare une solution concentrée d'au moins un sel minéral du métal ou des métaux que l'on désire obtenir sous forme d'oxalate, dans un milieu liquide contenant au moins 25% en volume d'un second solvant organique choisi parmi le méthanol, l'éthanol, le tétrahydrofuranne, et les polyols liquides à la température ambiante, ou des mélanges de ces solvants, le reste éventuel dudit milieu liquide étant constitué par de l'eau, la concentration totale des sels étant supérieure à 1 mole/litre, ladite solution étant acidifiée pour favoriser la solubilisation des sels et contenanat en outre les agents dopants éventuels;

puis que l'on verse progressivement ladite solution de sel dans ladite solution d'acide oxalique en agitant cette dernière, pour obtenir un précipité particulaire d'oxalate;

étant entendu que l'un au moins desdits solvants organiques est un alcool.

11. Procédé selon la revendication 10, caractérisé par le fait que ledit premier solvant organique est choisi parmi l'éthanol, le propanol-1, l'isopropanol, le butanol, l'acétone, l'éther éthylique et les mélanges éther de pétrol/éthanol.

12. Procédé selon l'une quelconque des revendications 10 à 11, caractérisé par le fait que l'on agite la solution d'acide oxalique et que l'on introduit progressivement la solution de sels dans ladite solution agitée en pulvérisant la solution de sels minéraux au-dessus de la solution d'acide oxalique.

13. Procédé selon l'une quelconque des revendications 10 à 12, caractérisé par le fait que ledit polyol est choisi parmi l'éthylèneglycol, le glycérol, le diéthylèneglycol et le propylèneglycol.

14. Procédé selon la revendication 13, caractérisé par le fait que l'on utilise ledit polyol en mélange avec l'eau et/ou avec un alcanol inférieur.

15. Procédé selon l'une quelconque des revendications 10 à 13, caractérisé par le fait que l'on réalise la solution de sels minéraux de départ en utilisant comme solvant:

les mélanges propanol-méthanol;

l'éthanol à 10% d'eau;

les mélanges propanediol-eau;

les mélanges tétrahydrofuranne-eau;

des mélanges eau-éthylèneglycol contenant au moins 25% d'éthylèneglycol, et l'éthylèneglycol pur;

des mélanges contenant au moins 20% d'éthylèneglycol et au moins 20% d'un autre polyol ou de méthanol, le reste étant de l'eau;

des mélanges eau-méthanol contenant au moins 50% de méthanol.

16. Procédé selon l'une quelconque des revendications 10 à 15, caractérisé par le fait que l'on règle la dimension des particules par le choix des solvants, les dimensions des particules étant d'autant plus réduites que la constante diélectrique des solvants organiques est plus faible et que l'eau est absente ou présente en faibles quantités.

17. Utilisation des compositions particulaires d'oxalate de métaux ferromagnétiques telles que définies dans l'une quelconque des revendications 1 à 9, caractérisée par le fait que l'on transforme lesdites particules, après addition éventuelle de dopants, en particules aciculaires d'oxydes, de nitrures, d'oxynitrures, de borures, de carbonitrures, d'oxycarbonitrures, de métaux ou d'alliages métalliques correspondants.

**Patentansprüche**

1. Teilchenförmige Zusammensetzungen von ferromagnetischen einfachen oder gemischten Metalloxalaten, dadurch gekennzeichnet, daß sie in Form nadelartiger Teilchen, die eine Länge von 0,05 bis 0,5 µm aufweisen, vorhanden sind, wobei ein Mengenanteil von mehr als 60% der Teilchen eine Länge gleich der mittleren Länge ± 0,1 µm und ein Nadelartigkeitsverhältnis über 3 haben, wobei diese mittlere Länge zwischen 0,15 und 0,35 µm liegt.

2. Teilchenförmige Zusammensetzung nach Anspruch 1, dadurch gekennzeichnet, daß dieses Mengenverhältnis oberhalb 80% beträgt.

3. Teilchenförmige Zusammensetzung nach Anspruch 1, dadurch gekennzeichnet, daß das ferromagnetische Metall ausgewählt ist aus Eisen, Kobalt und Nickel.

4. Teilchenförmige Zusammensetzung nach einem der vorhergehenden Ansprüche, dadurch gekennzeichnet, daß das Oxalat ein gemischtes Oxalat ist, bestehend aus mindestens einem ferromagnetischen Metall, mindestens einem zweiwertigen Metall, ausgewählt aus Kupfer, Zink, Cadmium, Magnesium, Mangan, den Erdalkalimetallen, (Calcium, Barium, Strontium), Europium, Ytterbium und Blei.

5. Teilchenförmige Zusammensetzung nach den vorhergehenden Ansprüchen, dadurch gekennzeichnet, daß die Oxalate unter anderem als Adjuvanz oder Dotierungsmittel außerdem ein oder mehrere Elemente ausgewählt aus Lithium, Kalium, Aluminium, Scandium, Titan, Vanadium, Chrom, Yttrium, Zirkon, Niob, Molybdän, Wismuth, Arsen, Bor, Antimon, Silicium, Germanium, Gallium, Indium, den dreiwertigen Seltenen Erden (z.B. Gadolinium, Neodym, Dysprosium, Samarium, Terbium, Cer, Präseodym, Holmium, Erbium, Thulium, Lutetium), enthält.

6. Teilchenförmige Zusammensetzung nach Anspruch 5, dadurch gekennzeichnet, daß das Dotierungsmittel in einer Menge unter 3 Gew.-% vorhanden ist.

7. Teilchenförmige Zusammensetzung nach den vorhergehenden Ansprüchen, dadurch gekennzeichnet, daß das Oxalat ein Oxalat auf Basis von Eisen ist, das mindestens 60% Eisanatome im Verhältnis zur Gesamtzahl der Metallatome aufweist.

8. Teilchenförmige Zusammensetzung nach den vorhergehenden Ansprüchen, dadurch gekennzeichnet, daß die Oxalate (abgesehen von gegebenenfalls vorliegenden Dotierungsmitteln) der Formel entsprechen:

$$(Fe_{1-x}M_x)C_2O_4,\ y\ H_2O$$

worin

M mindestens ein Metall, ausgewählt aus Kobalt, Nickel, Kupfer, Zink, Cadmium, Magnesium, Mangan, den Erdalkalimetallen, Europium, Ytterbium, und Blei darstellt,

y eine Zahl über oder gleich Null und unter oder gleich zwei ist und

x eine Zahl unter oder gleich 1/3 ist.

9. Teilchenförmige Zusammensetzung nach Anspruch 8, dadurch gekennzeichnet, daß x unterhalb 0,15 beträgt.

10. Verfahren zur Herstellung einer teilchenförmigen Zusammensetzung, die definiert ist durch die vorhergehenden Ansprüche, dadurch gekennzeichnet, daß:

man einerseits eine Lösung herstellt, die 0,1 bis 1 Mol je Liter Oxalsäure in einem ersten organischen Lösungsmittel mit einer Dielektrizitätskonstante unter 30 enthält, wobei diese Lösung maximal 50 Vol.-% Wasser enthalten kann;

man andererseits eine konzentrierte Lösung von mindestens einem Mineralsalz eines Metalls oder von Metallen, die man in Form des Oxalats zu erhalten wünscht, in einem flüssigen Milieu, das mindestens 55 Vol.-% eines zweiten organischen Lösungsmittels, ausgewählt aus Methanol, Ethanol, Tetrahydrofuran und Polyolen, enthält, die bei Umgebungstemperatur flüssig sind, oder von Mischungen dieser Lösungsmittel, wobei der gegebenenfalls vorhandene Rest des flüssigen Milieus durch Wasser gebildet wird, wobei die Gesamtkonzentration der Salze oberhalb 1 Mol/l beträgt und die Lösung angesäuert ist, um die Solubilisierung der Salze zu begünstigen, und sie außerdem gegebenenfalls Dotierungsmittel enthält, herstellt; und daß man danach fortschreitend die Salzlösung in die Oxalsäurelösung gießt, wobei man letztere rührt, um einen teilchenförmigen Oxalataniederschlag zu erhalten; mit der Maßgabe, daß mindestens eines dieser organischen Lösungsmittel ein Alkohol ist.

11. Verfahren nach Anspruch 10, dadurch gekennzeichnet, daß dieses erste organische Lösungsmittel ausgewählt ist aus Ethanol, Propanol-1, Isopropanol, Butanol, Aceton, Ethylether und Gemische von Petrolether/Ethanol.

12. Verfahren nach den Ansprüchen 10 und 11, dadurch gekennzeichnet, daß man die Oxalsäurelösung rührt und daß man beim fortschreitenden Leiten der Salzlösung in diese gerührte Lösung die Mineralsalzlösung oberhalb der Oxalsäurelösung zerstäubt.

13. Verfahren nach den Ansprüchen 10 bis 12, dadurch gekennzeichnet, daß das Polyol ausgewählt ist aus Ethylenglykol, Glycerol, Diethylenglykol und Propylenglykol.

14. Verfahren nach Anspruch 13, dadurch gekennzeichnet, daß man das Polyol in Mischung mit Wasser und/oder mit einem niederen Alkanol anwendet.

15. Verfahren nach den Ansprüchen 10 bis 13, dadurch gekennzeichnet, daß man die Lösung der Mineralsalze so herstellt, daß man als Lösungsmittel die folgenden verwendet:

Mischungen von Propanol-Methanol;

Ethanol mit 10% Wasser;

Mischungen von Propandiol-Wasser;

Mischungen von Tetrahydrofuran-Wasser;

Mischungen von Wasser-Ethylenglykol, die mindestens 25% Ethylenglykol enthalten, und reines Ethylenglykol;

Mischungen, die mindestens 20% Ethylenglykol und mindestens 20% von einem anderen Polyol oder von Methanol enthalten und der Rest Wasser ist;

Mischungen aus Wasser-Methanol, die mindestens 50% Methanol enthalten.

16. Verfahren nach den Ansprüchen 10 bis 15, dadurch gekennzeichnet, daß man die Teilchendimension durch Auswahl der Lösungsmittel einstellt, wobei die Teilchendimensionen um so mehr reduziert sind, je mehr die Dielektrizitätskonstante der organischen Lösungsmittel schwächer ist und Wasser abwesend oder in geringen Mengen anwesend ist.

17. Verwendung von teilchenförmigen Zusammensetzungen von ferromagnetischen Metalloxalaten, wie sie in den Ansprüchen 1 bis 9 definiert worden sind, dadurch gekennzeichnet, daß man die Teilchen gegebenenfalls nach Zugabe von Dotierungsmitteln, in nadelförmige Teilchen der entsprechenden Oxide, Nitride, Oxynitride, Boride, Carbonitride, Oxycarbonitride, Metalle oder Metallegierungen überführt.

## Claims

1. A particulate composition of simple or mixed oxalate of ferromagnetic metals, characterized by the fact that it appears in the form of acicular particles having a length of from 0.05 to 0.5 micrometres, with a proportion of more than 60% of the particles having a high length equal to the average length $\pm$ 0.1 µm and an acicular ratio higher than 3, the said average length lying between 0.15 and 0.35 µm.

2. A particulate composition as in Claim 1, characterized by the fact that the said proportion is higher than 80%.

3. A particulate composition as in Claim 1, characterized by the fact that the ferromagnetic metal is chosen from iron, cobalt and nickel.

4. A particulate composition as in any one of the preceding Claims, characterized by the fact that the said oxalate is a mixed oxalate comprising besides at least one ferromagnetic metal at least one bivalent metal chosen from copper, zinc, cadmium, magnesium, manganese, the alkaline earth metals (calcium,

barium, strontium), europium, ytterbium, tin and lead.

5. A particulate composition as in any one of the preceding Claims, characterized by the fact that the said oxalate contains in addition by way of adjuvant or doping agent one or more elements chosen from lithium, potassium, aluminium, scandium, titanium, vanadium, chromium, yttrium, zirconium, niobium, molybdenum, bismuth, arsenic, boron, antimony, silicon, germanium, gallium, indium, the trivalent rare earths (for example, gadolinium, neodymium, dysprosium, samarium, terbium, cerium, praseodymium, holmium, erbium, thulium, lutetium).

6. A particulate composition as in Claim 5, characterized by the fact that the said doping agent is present in a proportion of less than 3% by weight.

7. A particulate composition as in any of the preceding Claims, characterized by the fact that the said oxalate is an iron-base oxalate containing at least 60% of iron atoms in proportion to the whole of the metallic atoms.

8. A particulate composition as in any one of the preceding Claims, characterized by the fact that the said oxalate corresponds (apart from the doping agents if any) with the formula:

$$(Fe_{1-x}M_x)\ C_2O_4,\ y\ H_2O$$

in which:

M represents at least one metal chosen from cobalt, nickel, copper, zinc, cadmium, magnesium, manganese, the alkaline earth metals, europium, ytterbium, tin and lead;

y is a number higher than or equal to zero and less than or equal to 2; and x is a number less than or equal to 1/3.

9. A particulate composition as in Claim 8, characterized by the fact that x is less than 0.15.

10. A method of preparation of a particulate composition as defined in any of the preceding Claims, characterized by the fact that:

firstly one prepares a solution containing from 0.1 to 1 mole per litre of oxalic acid in a first organic solvent having a dieletric constant less than 30, whilst the said solution may contain a maximum of 15% by volume of water;

secondly one prepares a concentrated solution of at least one mineral salt of the metal or metals which it is desired to obtain in the form of oxalate, in a liquid medium containing at least 25% by volume of a second organic solvent chosen from methanol, ethanol, tetrahydrofuran and the polyols liquid at the ambient temperature, or mixtures of these solvents, the rest if any of the said liquid medium consisting of water, the total concentration of the salts being higher than 1 mole per litre, the said solution being acidified in order to assist in rendering the salts soluble and containing in addition the doping agents if any;

then one progressively pours the said solution of salt into the said solution of oxalic acid while agitating the latter, in order to obtain a particulate precipitate of oxalate; it being understood that at least one of the said organic solvents is an alcohol.

11. A method as in Claim 10, characterized by the fact that the said first organic solvent is chosen from ethanol, propanol-1, isopropanol butanol, acetone, ethyl ether and the petroleum/ethanol ether mixtures.

12. A method as in either of the Claims 10 to 11, characterized by the fact that one agitates the solution of oxalic acid and that one introduces the solution of salts progressively into the said agitated solution by atomizing the solution of mineral salts above the solution of oxalic acid

13. A method as in any one of the Claims 10 to 12, characterized by the fact that the said polyol is chosen from ethylene glycol, glycerol, diethylene glycol and propylene glycol.

14. A method as in Claim 13, characterized by the fact that the said polyol is employed in a mixture with water and/or with a lower alkanol.

15. A method as in any one of the Claims 10 to 13, characterized by the fact that the original solution of mineral salts is realized by employing as solvent:

propanol-methanol mixtures;

ethanol with 10% of water;

propanediol-water mixtures;

tetrahydrofuran-water mixtures;

water-ethylene glycol mixtures containing at least 25% of ethylene glycol, and pure ethylene glycol;

mixtures containing at least 20% of ethylene glycol and at least 20% of another polyol or methanol, the rest being water;

water-methanol mixtures containing at least 50% of methanol.

16. A method as in any of the Claims 10 to 15, characterized by the fact that the dimensions of the particles are regulated by the choice of solvents, the dimensions of the particles being smaller the lower the dielectric constant of the organic solvents and according to whether water is absent or present in small amounts.

17. The employment of particulate compositions of oxalates of ferromagnetic metals, as defined in any one of the Claims 1 to 9, characterized by the fact that after the possible addition of doping agents the said particles are transformed into acicular particles of oxides, nitrides, oxynitrides, borides, carbonitrides or oxycarbonitrides of corresponding metals or metallic alloys.

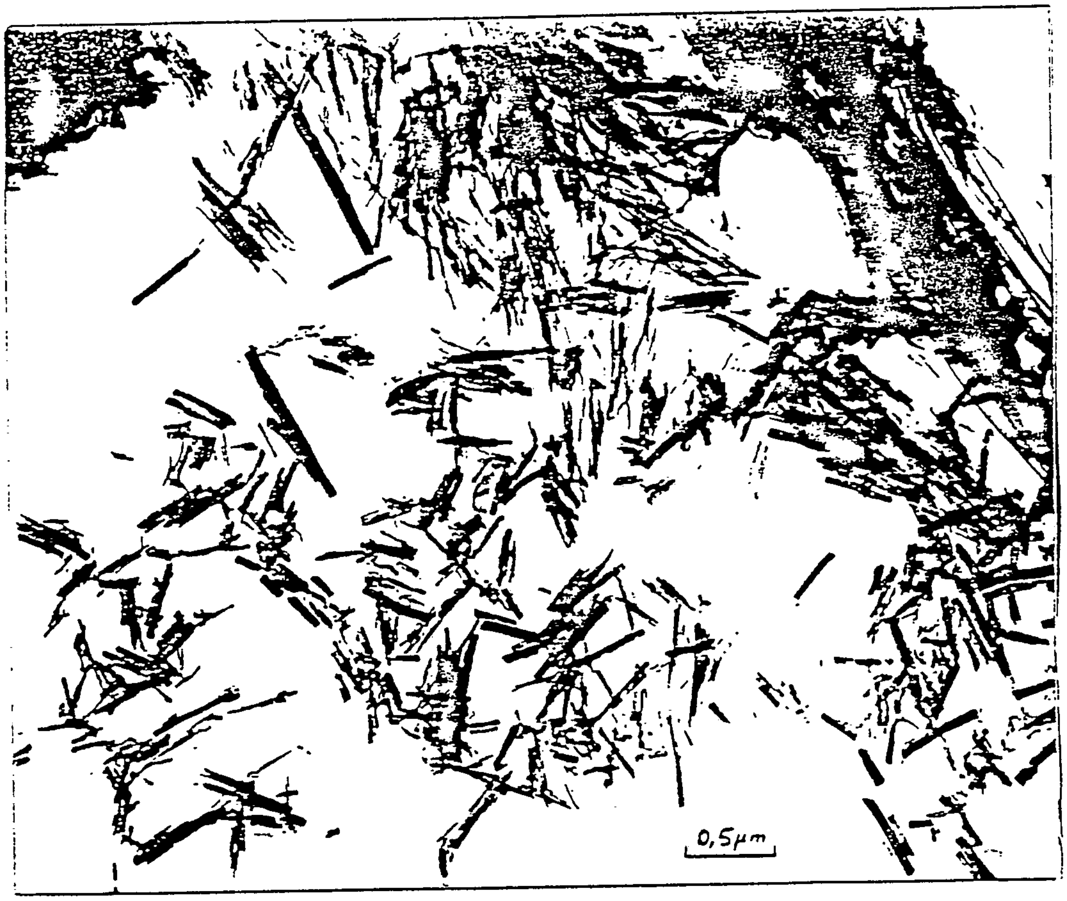


Figure 1

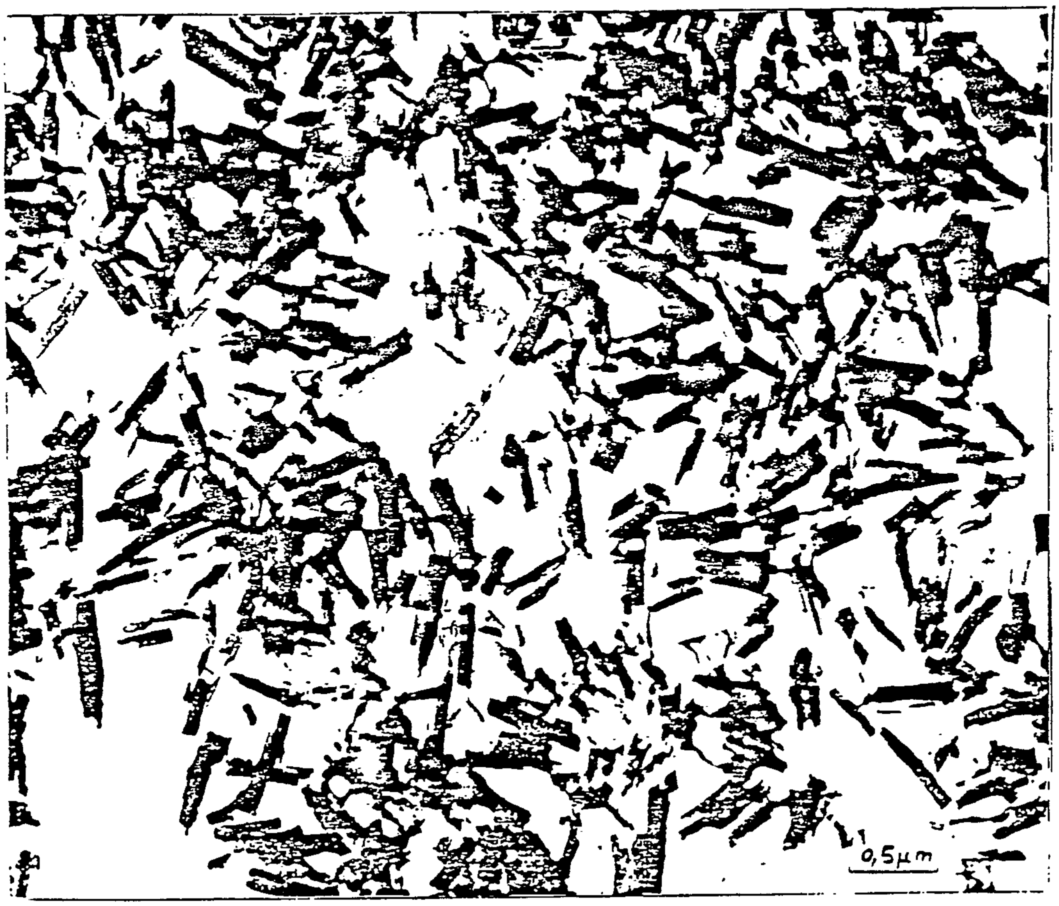


Figure 2